(19) Europäisches Patentamt
European Patent Office
Office européen des brevets

(11) Publication number: **0 444 764 A1**

# (12) EUROPEAN PATENT APPLICATION

(21) Application number: **91250051.9**

(51) Int. Cl.[5]: **C07D 417/04**, C07D 277/68

(22) Date of filing: **22.02.91**

(30) Priority: **27.02.90 DE 4006555**

(43) Date of publication of application:
**04.09.91 Bulletin 91/36**

(84) Designated Contracting States:
**AT BE CH DE DK ES FR GB GR IT LI LU NL SE**

(71) Applicant: **SCHERING AKTIENGESELLSCHAFT**
**Müllerstrasse 170/178**
**W-1000 Berlin 65(DE)**

(72) Inventor: **Dorfmeister, Gabriele, Dr.**
**Heiligenseestrasse 70**
**W-1000 Berlin 27(DE)**
Inventor: **Franke, Wilfried, Dr.**
**Spiessergasse 6b**
**W-1000 Berlin 27(DE)**
Inventor: **Ganzer, Michael, Dr.**
**Eichborndam 279**
**W-1000 Berlin 26(DE)**

## (54) Process and intermediates for the preparation of substituted 1-benzothiazolyl-3,4-dimethyl-3-pyrrolin-2,5(1H)-diones.

(57) The invention relates a new process for the preparation of 1-benzothiazolyl-3,4-dimethyl-3-pyrrolin-2,5(1H)-diones of general formula I

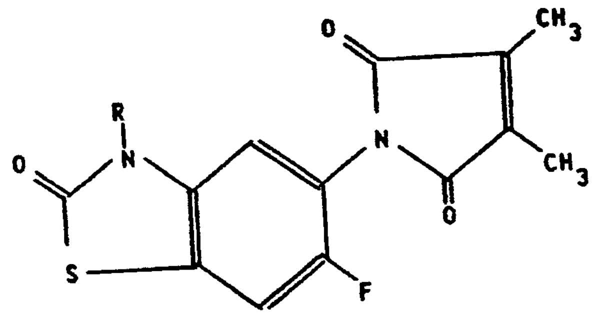

(I) ,

in which R is $C_1$-$C_6$-alkyl, $C_2$-$C_6$-alkenyl or $C_3$-$C_6$-alkynyl. which starts from the known halobenzothiazole of general formula II

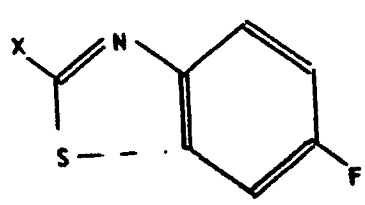

(II) ,

in which X is chlorine or bromine, via new alkoxy-, alkenyloxy- or alkynyloxybenzothiazoles of general formula IV

EP 0 444 764 A1

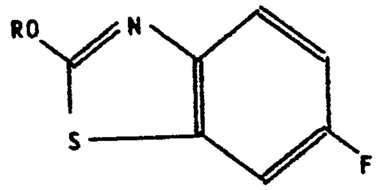

(IV) ,

via new nitrobenzothiazoles of general formula V

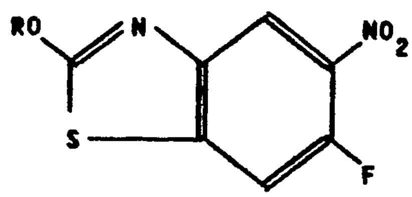

(V) ,

via new aminobenzothiazoles of general formula VI

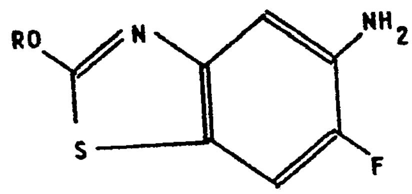

(VI) ,

and via new imides of general formula VIII.

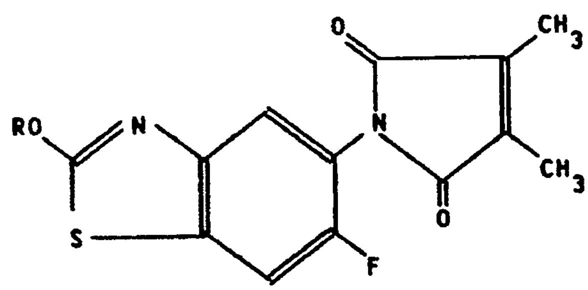

(VIII) ,

This invention relates to a new process and new intermediates for the preparation of substituted 1-benzothiazolyl-3,4-dimethyl-3-pyrroline-2,5(1H)-diones of general formula I

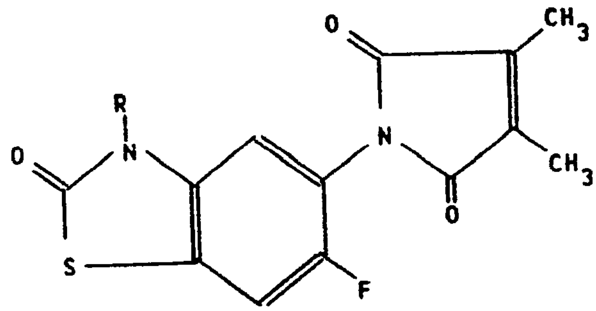

(I) ,

I in which R is $C_1$-$C_6$-alkyl, $C_2$-$C_6$-alkenyl or $C_3$-$C_6$-alkynyl.

These compounds have excellent herbicidal activity against a broad spectrum of monocotyledonous and dicotyledonous weeds in agricultural crops. Their preparation and use are described in EP 311 135. The object of the present invention is to provide a new process which allows preparation of compounds of general formula I without any problems, and under mild reaction conditions.

This object is solved by a process for the preparation of 1-benzothiazolyl-3,4-dimethyl-3-pyrrolin-2,5-(1H)-diones in which

a) a halobenzothiazole of general formula II

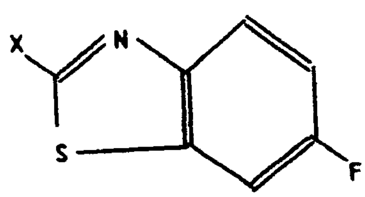

(II) ,

in which X is chlorine or bromine is reacted with an alkali metal alcoholate of general formula III

R-OMe (III)

in which R has the meaning given under general formula I and Me is sodium or potassium, in a suitable solvent.

b) the alkoxy-, alkenyloxy- or alkynyloxybenzothiazole, so formed, of general formula IV

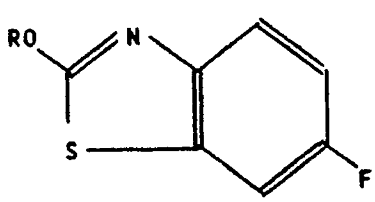

(IV) ,

in which R has the meaning given under general formula I, is nitrated with nitric acid in a suitable solvent,

c) the nitrobenzothiazole, so formed, of general formula V

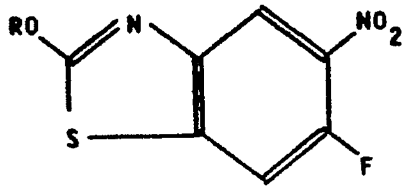

(V) ,

in which R has the meaning given under general formula I, is reduced in a suitable solvent,
d) the aminobenzothiazole, so formed, of general formula VI

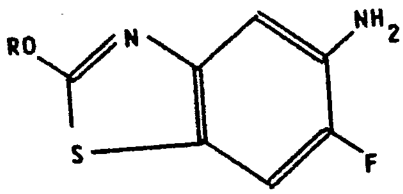

(VI) ,

in which R has a meaning given under general formula I, is treated with dimethylmaleic anhydride of general formula VII

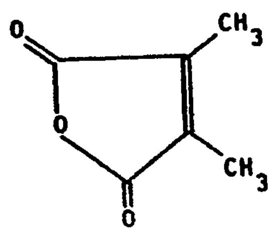

(VII) ,

in a suitable solvent, and finally
e) the imide, so formed, of general formula VIII

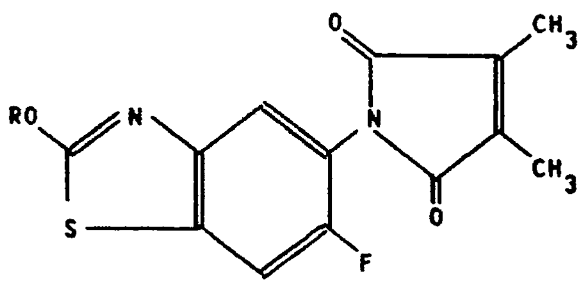

(VIII) ,

in which R has the meaning given under general formula I is rearranged with a quaternary ammonium salt in a suitable solvent.

The compounds prepared in the individual reaction stages can optionally be present in various enantiomers and these fall within the scope of the invention.

The halobenzothiazole of general formula II that is used as the starting material for reaction stage a) can be obtained by diazotisation of an aminobenzothiazole and then by treatment with a mineral acid, as described for example in J. Indian Chem. Soc. 10 (1933) 465. The synthesis of the corresponding aminobenzothiazole can be carried out by treatment of 4-fluoroaniline with an isocyanate and an oxidising agent (Liebigs Ann. Chem. 67 (1934) 944).

The alkoxy-, alkenyloxy- or alkynyloxybenzothiazoles used as starting materials for reaction stage b), of general formula IV

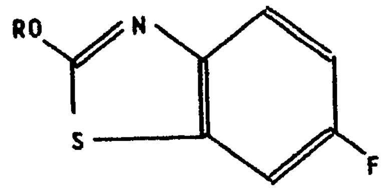

(IV) ,

in which R is $C_1$-$C_6$-alkyl, $C_2$-$C_6$-alkenyl or $C_3$-$C_6$ alkynyl, are new and also fall within the scope of the invention. They can be prepared from the halobenzothiazoles of general formula II by known method. Treatment with sodium in the corresponding alcohol has been shown to be suitable. The reaction time is from 0.5 to 12 hours and the reaction temperature lies within the range of 0 °C to the boiling point of the alcohol used.

The nitrobenzothiazoles used as starting materials for reaction stage c) of general formula V

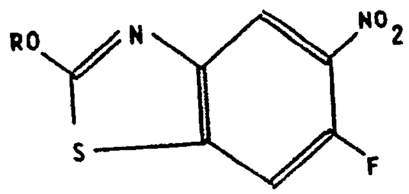

(V) ,

in which R is $C_1$-$C_6$-alkyl, $C_2$-$C_6$-alkenyl or $C_3$-$C_6$-alkynyl, are new and also fall within the scope of the invention. They can be prepared from the alkoxy, alkenyloxy or alkynyloxybenzothiazoles of general formula IV by known methods. Suitably the reaction is carried out with nitric acid in sulphuric acid. The reaction time is generally from 0.5 to 12 hours and the reaction temperature lies in the range of -10 °C to 0 °C.

Surprisingly in the present case the nitration occurs mainly in the desired 5 position and not in the 7 position as would have been expected from J. Org Chem 18 (1953) 1092.

Also in the present case threefold nitration does not occur as would be expected to happen according to Houben-Weyl, Band X/1 page 96, but instead compounds are obtained with nitration in the 5 position.

The aminobenzothiazoles used as the starting materials in reaction stage d) of general formula VI

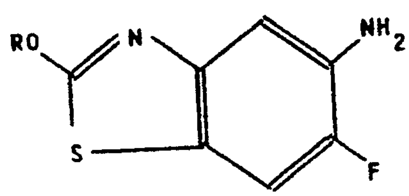

(VI) ,

in which R is $C_1$-$C_6$-alkyl, $C_2$-$C_6$-alkenyl or $C_3$-$C_6$-alkynyl, are also new and also fall within the scope of the invention. They can be obtained from the nitrobenzothiazoles of general formula V by known methods. Suitably the reduction can be carried out with iron in a solvent mixture of ethyl acetate/5% acetic acid/glacial acetic acid. The reaction time is from 0.5 to 12 hours and the reaction temperature lies within the range of 20 °C up to the boiling point of the solvent.

The imides used as starting materials for reaction stage e) of general formula VIII

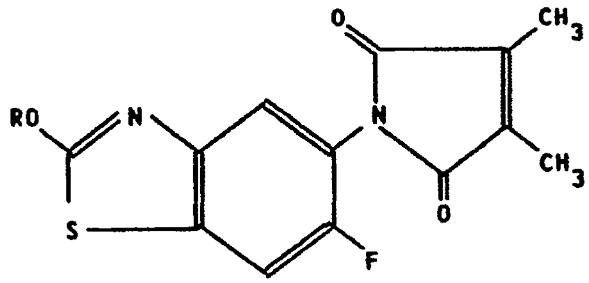

(VIII) ,

in which R is $C_1$-$C_6$-alkyl, $C_2$-$C_6$-alkenyl or $C_3$-$C_6$-alkynyl, are also new and fall within the scope of the invention. They can be prepared by known methods from aminobenzothiazoles of general formula VI and dimethylmaleic anhydride of general formula VII. Suitably the reaction is carried out in acetic acid. The reaction time is from 0.5 to 15 hours and the reaction temperature lies within the range of 20 °C up the boiling point of the solvent.

The imides of general formula VIII can be rearranged with a quaternary ammonium salt as catalyst in a suitable solvent to the heterocyclic substituted azoles of general formula I. The reaction is suitably carried out by treating the imide of general formula VIII in toluene with the addition of tetrabutylammonium bromide. The reaction time is from 0.5 to 12 hours and the reaction temperature lies in the range of 20 °C to the boiling point of the solvent.

Suitable catalysts include tertiary amines, bicyclic bases, such as 1,8-diazabicyclo[5.4.0]undec-7-ene (DBU) and 1,5-diazabicyclo[4.3.0]non-5-ene (DBN), as well as dialkyl sulphates.

Suitable solvents for all reaction stages include hydrocarbons, such as for example toluene, chlorinated hydrocarbons such as for example methylene chloride or chloroform, ethers such as for example diethyl ether, dioxane or tetrahydrofuran, alcohols, such as for example methanol or ethanol, ketones, such as for example acetone or butanone, amides, such as for example dimethyl formamide and also sulphoxides, such as for example dimethyl sulphoxide.

The invention is illustrated in the following examples.

Example 1 - Reaction Stage a)

6-Fluoro-2-(2-propynyloxy)benzothiazole

2 g Sodium was added portionwise with ice-cooling to 90 ml propargyl alcohol at such a rate that the temperature does not rise above 40 °C. After all the sodium had dissolved, the mixture was cooled to 0 °C and treated with 16.1g 2-chloro-6-fluorobenzothiazole. The ice bath was removed and mixture heated with stirring to reflux. It was then refluxed for 40 minutes. After cooling the reaction mixture was concentrated under reduced pressure. The residue was treated with diisopropyl ether, filtered and the precipitate washed with diisopropyl ether. The combined filtrate was concentrated and the residue recrystallised from hexane in a small amount of diisopropyl ether.

Yield: 13.2g = 74% of theory
mp: 63 °C

Example 2 - Reaction Stage b)

6-Fluoro-5-nitro-2-(2-propynyloxy)benzothiazole

10.4 g 6-Fluoro-2-(2-propynyloxy)benzothiazole was dissolved in 100 ml concentrated sulphuric acid and cooled to -10 °C. At this temperature, 2.5 ml 100% nitric acid was added dropwise. The mixture was stirred for one hour at -10 °C and then poured into 400 ml ice. The aqueous phase was shaken several times with acetic acid, the combined organic phases washed with sodium chloride, dried and concentrated. The crude product was purified by column chromatograph with hexane/ethyl acetate = 9:1.

Yield: 9.5g = 75% of theory
mp: 101 °C

Example 3 - Reaction Stage c)

5-Amino-6-fluoro-5-nitro-2-(2-propynyloxy)benzothiazole

8.3 g Iron powder was added to 25 ml 5% aqueous acetic acid, the mixture heated to 80° C and under strong stirring a solution of 8.2 g 6-fluoro-5-nitro-2-(2-propynyloxy)benzothiazole in 30 ml ethyl acetate and 30 ml acetic acid was added. The mixture was heated for 2 hours under reflux. After cooling, the mixture was diluted with ethylacetate and water and filtered over celite. The solid was washed several times with ethyl acetate, the phases were separated, the organic phase washed with aqueous sodium chloride, dried over magnesium sulphate and concentrated. The residue was recrystallised from hexane diisopropyl ether.

Yield: 4.4g = 61% of theory
mp: 140° C

Example 4 - Reaction Stage d)

1-[6-Fluoro-2-(2-propynyloxy)-5-benzothiazolyl]-3,4-dimethyl-3-pyrroline-2,5(1H)-dione

3.3 g 5-Amino-6-fluoro-5-nitro-2-(2-propynyloxy)benzothiazole and 2,3-dimethylmaleic anhydride was dissolved in 50 ml acetic acid and the mixture heated for 5 hours at reflux. The cooled reaction mixture was concentrated, the residue taken up in ether and separated from undissolved material. The filtrate was concentrated again. The residue was stirred with isopropylether, filtered and washed with isopropylether.

Crude Yield: 3 g = 60% pf theory

The crude product purified by several recrystallisations from diisopropylether.

Yield: 2 g = 40% of theory
mp: 151° C

Example 5 - Reaction Stage e)

1-[6-Fluoro-2-oxo-3-(2-propynyl)-2,3-dihydro-5-benzothiazolyl]-3,4-dimethyl-3-pyrroline-2,5(1H)-dione

200 mg 1-[6-Fluoro-2-(2-propynyloxy)-5-benzothiazolyl]-3,4-dimethyl-3-pyrroline-2,5(1H)-dione was dissolved in 5 ml toluene and 40 mg tetrabutylammonium bromide added. The mixture was heated for 24 hours under reflux, concentrated under reduced pressure, the residue triturated with ether/ethyl acetate, filtered and dried.

Yield: 140 g = 70% of theory
mp: 216-218° C

## Claims

1. A process for the preparation of substituted 1-benzothiazolyl-3,4-dimethyl-3-pyrrolin-2,5(1H)-diones of general formula I

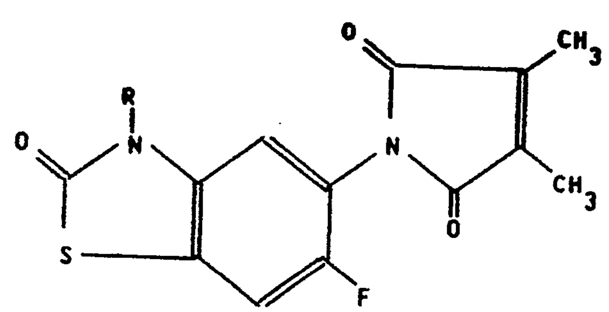

(I) ,

in which R is $C_1$-$C_6$-alkyl, $C_2$-$C_6$-alkenyl or $C_3$-$C_6$-alkynyl, wherein

a) a halobenzothiazole of general formula II

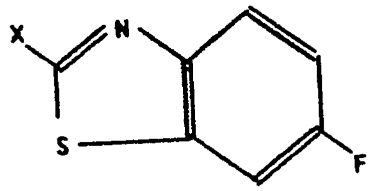

(II) ,

in which X is chlorine or bromine is reacted with an alkali metal alcoholate of general formula III

R-OMe (III)

in which R has the meaning given under general formula I and Me is sodium or potassium, in a suitable solvent.

b) the alkoxy-, alkenyloxy- or alkynyloxybenzothiazole, so formed, of general formula IV

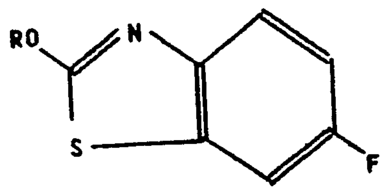

(IV) ,

in which R has the meaning given under general formula I, is nitrated with nitric acid in a suitable solvent,

c) the nitrobenzothiazole, so formed, of general formula V

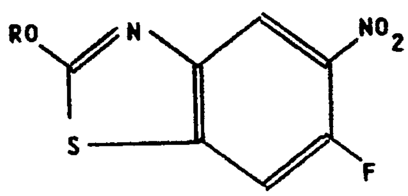

(V) ,

in which R has the meaning given under general formula I, is reduced in a suitable solvent,

d) the aminobenzothiazole, so formed, of general formula VI

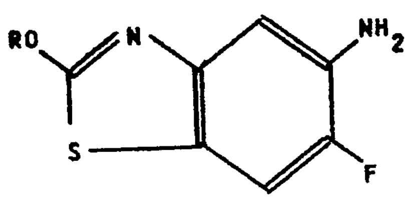

(VI) ,

in which R has a meaning given under general formula I, is treated with dimethylmaleic anhydride of general formula VII

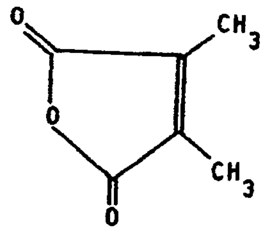

(VII) ,

in a suitable solvent, and finally

e) the imide, so formed, of general formula VIII

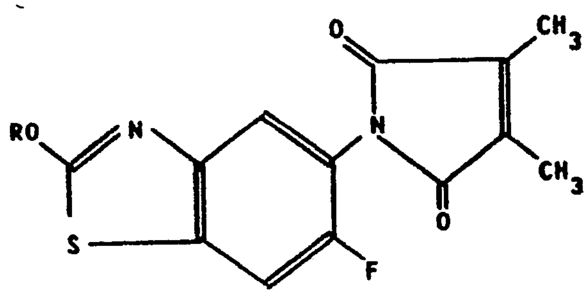

(VIII) ,

in which R has the meaning given under general formula I is rearranged with a quaternary ammonium salt in a suitable solvent.

2. Alkoxy-, alkenyloxy- or alkynyloxybenzothiazoles of general formula IV

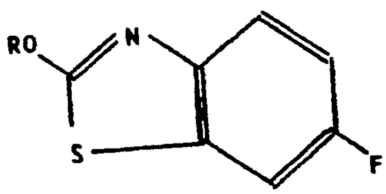

(IV) ,

in which R is $C_1$-$C_6$-alkyl, $C_2$-$C_6$-alkenyl or $C_3$-$C_6$-alkynyl.

3. Nitrobenzothiazoles of general formula V

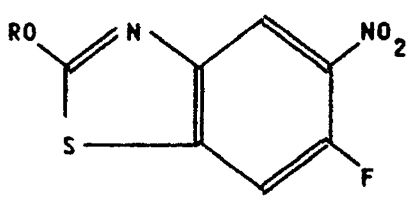

(V) ,

in which R is $C_1$-$C_6$-alkyl, $C_2$-$C_6$-alkenyl or $C_3$-$C_6$-alkynyl.

4. Aminobenzothiazoles of general formula VI

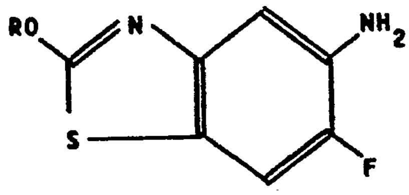

(VI) ,

in which R is $C_1$-$C_6$-alkyl, $C_2$-$C_6$-alkenyl or $C_3$-$C_6$-alkynyl.

**5.** Imides of general formula VIII

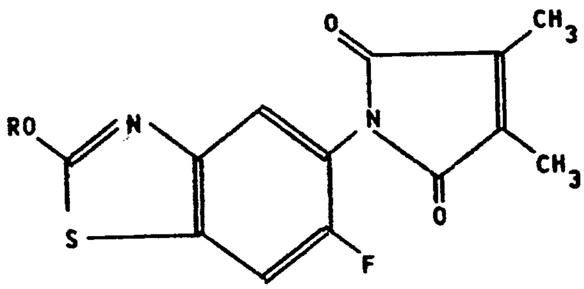

(VIII) ,

in which R is $C_1$-$C_6$-alkyl, $C_2$-$C_6$-alkenyl or $C_3$-$C_6$-alkynyl.

European Patent Office

# EUROPEAN SEARCH REPORT

Application Number

**EP 91 25 0051**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 113, no. 1, 2nd July 1990, page 662, abstract no. 23901k, Columbus, Ohio, US; & JP-A-02 04 784 (NIHON TOKUSHU NOYAKU SEIZO K.K.) 09-01-1990 * Abstract * | 2,3,4 | C 07 D 417/04<br>C 07 D 277/68 |
| X | EP-A-0 351 676 (NIHON TOKUSHU NOYAKU SEIZO) * Claims * | 1 | |
| D,X | EP-A-0 311 135 (SCHERING AG) * Claims * | 1 | |
| P,X | EP-A-0 373 461 (NIHON TOKUSHU NOYAKU SEIZO) * Claims; pages 11-13,29-30,41-47 * | 2-5 | |

TECHNICAL FIELDS SEARCHED (Int. Cl.5)

C 07 D 277/00
C 07 D 417/00

The present search report has been drawn up for all claims

| Place of search | Date of completion of search | Examiner |
|---|---|---|
| The Hague | 02 May 91 | HENRY J.C. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document